# EUROPEAN PATENT SPECIFICATION

(11) **EP 1 493 457 B1**
(45) Date of publication and mention of the grant of the patent: **19.11.2008**
(21) Application number: 04253985.8
(22) Date of filing: 01.07.2004
(51) Int. Cl.: A61L 31/16

(54) **Polymer-coated biomedical devices**
Polymer-beschichtete biomedizinische Artikel
Dispositif biomédicinal revêtu de polymère

(30) Priority: 01.07.2003 US 484197 P
(43) Date of publication of application: 05.01.2005
(73) Proprietor: Medtronic Vascular, Inc., Santa Rosa CA 95403 (US)
(72) Inventor: Moriarty, James W. Jr., Windsor, California (US)
(74) Representative: Marsden, John Christopher

(56) References cited:
- EP-A- 0 623 354
- WO-A-91/12779
- US-A1- 2003 109 647

## Description

### FIELD OF THE INVENTION

This invention relates generally to polymer-coated biomedical devices such as drug-polymer coated stents. More specifically it relates to the use of activatable adhesion promoters to enhance adhesion of the polymer coatings to such devices.

### BACKGROUND OF THE INVENTION

Drug-polymer coated stents typically contain one or more therapeutic compounds within a polymeric matrix disposed on a metallic stent to improve the efficacy of such endovascular stents. Thus, for example, EP-A-0 623 354 describes a method of manufacture of an intravascular stent wherein a generally cylindrical stent body is coated with a solution of a polymer and a therapeutic substance in a solvent which is thereafter evaporated. WO-A-9112779 discloses an intraluminal drug-eluting prosthesis constructed and arranged such that at least a portion of its exterior surface is formed from a polymer into which is compounded a drug capable of limiting acute or chronic luminal closure. US-A-2003/0109647 describes hydroxyl-terminated polyhydric alcohol esters which may be reacted, for example with maleic anhydride, to generate 2-carboxy ethenyl groups; the resulting products may be used in the formation of hydrogels for drug delivery, e.g. in the form of coating systems for vascular stents.

The therapeutic compounds are eluted from the stent coating after the drug-polymer stent is implanted in the body, delivering their patent effects in the tissue bed surrounding the implanted stent. The effectiveness of these drugs is generally improved because of delivery at the point of deployment. The localized levels of the medications may be higher and potentially more effective than orally or intravenously delivered drugs which distribute throughout the body and which may have minimal effect on the impacted area or may be expelled rapidly from the body without reaching their pharmaceutical intent. Drug release from tailored stent coatings may have controlled, timed-release qualities, eluting their bioactive agents over hours, weeks or even months.

For controlled delivery of the pharmaceutical compounds, polymer matrices containing the compounds must be reliably attached to the stent to maintain high quality during manufacturing of such a stent, and to prevent cracking or flaking of the drug-polymer coating when the stent is deployed. The coating may crack or fall off during assembly, packaging, storage, shipping, preparation and sterilization prior to deployment unless effectively adhered to the stent framework. Degradation of the polymer coating may occur with prolonged exposure to light and air, as the constituents of the drug polymer may oxidize or the molecular chains may scission. Although degradation of the polymer coating is of major concern, it is imperative that the adhesion strength of the coating be greater than the cohesive strength of the polymeric matrix to avoid loss of the coating.

Polymeric coatings have a tendency to peel or separate from an underlying metallic stent due to low adhesion strength typical between polymers and metals. Many polymers are non-polar or have limited polarization, reducing their ability to stick to the metal stent framework. Temperature excursions of the coated stent and the difference in thermal expansion coefficients between the metal and the coating may contribute to the fatigue and failure of the bond. Materials that are optimum for drug compatibility and elution may not, in and of themselves, provide sufficient adhesion to a metal substrate. A method to improve the adhesion between a drug-polymer coating and a metallic stent, while retaining the therapeutic characteristics of the drug-polymer stent, would be beneficial. Conventional polymers could be used in the drug-polymer coating. If the adhesive strength of the polymeric coating were improved, a more robust device could be made with lower profiles and the stent struts could touch. Other aspects of the drug-polymer coating, such as cohesive strength, hardness, creep resistance, bulk failure mechanisms and extent of deformation, may impact the robustness of the drug-polymer stent.

It is desirable, therefore, to provide a drug-polymer coated stent with improved adhesion between the drug polymer and the underlying stent framework, to provide a method for improving the adhesion of a polymer coating to a metallic stent, to provide a system for treating heart disease and other vascular conditions using drug-eluting stents with improved adhesion between the drug polymer and the stent, and to overcome the deficiencies and limitations described above.

### SUMMARY OF THE INVENTION

One aspect of the invention provides a polymer-coated biomedical device in which an activated adhesion promoter comprising a ring-opened copolymer of maleic anhydride and an olefin is present within the polymer coating or between the coating and the device.

Another aspect of the invention includes a method for preparing a polymer-coated biomedical device in which an activatable adhesion promoter comprising a copolymer of maleic anhydride and an olefin is incorporated into the polymeric coating or between the coating and the device and is thereafter activated to effect ring opening of the copolymer.

The biomedical device may advantageously be a drug-polymer coated stent, in particular such a stent having a metallic framework. Embodiments of such drug-polymer coated stents, which may if desired be coupled to a catheter to provide a system for treating a vascular system, are described in the following detailed description of the presently preferred embodiments, read in conjunction with the accompanying drawings.

Other implantable and blood-contacting biomedical devices in accordance with the invention include coated pacemaker leads, microdelivery pumps, feeding and delivery catheters, heart valves, artificial livers and other artificial organs.

### BRIEF DESCRIPTION OF THE DRAWINGS

**FIG. 1** is an illustration of one embodiment of a system for treating a vascular condition including a catheter, a stent, a drug-polymer coating, and an energy-activated adhesion layer, in accordance with the current invention;
**FIG. 2** is an illustration of a stent cross-section with a drug polymer and an activated adhesion promoter on the stent surface, in accordance with the current invention;
**FIG. 3** is an illustration of a stent cross-section with a drug-polymer coating on the stent surface with an energy-activated adhesion coating between the drug-polymer coating and the stent framework, in accordance with the current invention;
**FIG. 4** is a flow diagram of one embodiment of a method for manufacturing a drug-polymer coated stent with an activated adhesion promoter, in accordance with the current invention; and
**FIG. 5** is a flow diagram of one embodiment of a method for manufacturing a drug-polymer stent with an energy-activated adhesion coating and a drug-polymer coating, in accordance with the current invention.

### DETAILED DESCRIPTION OF THE

### PRESENTLY PREFERRED EMBODIMENTS

The adhesion of drug-polymer coatings to metallic stents may be improved by the inclusion of an activatable adhesion promoter comprising a copolymer of maleic anhydride and an olefin (e.g. ethylene) in a polymeric coating disposed on the stent. An activation process may be used to effect ring opening of the adhesion promoter, increasing the adhesion strength, and thereby improving the adhesion of the coating. An adhesion layer comprising the adhesion promoter may be applied to the stent and activated, followed by the drug polymer being applied. Alternatively, a drug polymer may be mixed with the adhesion promoter and applied to the stent, and the adhesion promoter then activated for improved adhesion.

One aspect of the present invention is a system for treating coronary heart disease and other vascular conditions, using catheter-deployed endovascular stents with polymeric coatings that include one or more drugs with desired timed-release properties and an adhesion promoter or an adhesion layer. Treatment of vascular conditions may include the prevention or correction of various ailments and deficiencies associated with the cardiovascular system, urinogenital systems, biliary conduits, abdominal passageways and other biological vessels within the body.

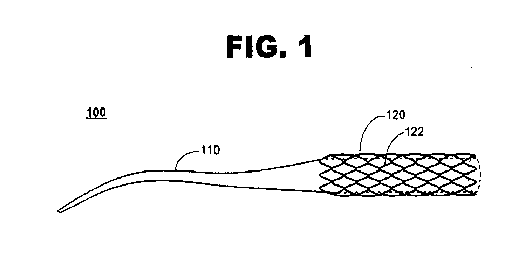

One embodiment of a system for treating a vascular condition, in accordance with the present invention, is illustrated in **FIG. 1** at **100**. Vascular condition treatment system **100** may include a catheter **110**, a stent **120** coupled to the catheter, and a drug-polymer coating **122** with an activated adhesion promoter or an underlying adhesion coating comprising such a promoter on the stent or stent framework.

The stent is coupled to the catheter, and may be deployed, for example, by pressurizing a balloon coupled to the stent or by retracting a sheath that allows the stent to expand to a prescribed diameter. The stent includes a stent framework. The stent framework may be formed from a base metal such as stainless steel, nitinol, tantalum, MP35N or other biocompatible alloy, platinum or titanium.

An adhesion layer comprising the activatable adhesion promoter may be disposed on the stent framework. Ring opening of the maleic anhydride component of the adhesion promoter provides additional polarization of the molecule and increases the bond energy, thus increasing the adhesion to the metallic stent material. The ethylene or other olefin copolymerised with the maleic anhydride therefore has a higher adhesion energy and improved adhesion to the metallic stent material.

A drug polymer may be disposed on the stent framework. The drug polymer may be mixed with the adhesion promoter and applied, or the drug polymer may be applied to the stent after an adhesion layer comprising the activatable adhesion promoter is disposed on the stent framework and activated. The adhesion of the drug polymer to a stent coated with such a polymeric adhesion layer would be enhanced because the drug polymer would essentially be coating over similar material. The adhesion promoter may be activated prior to or after coating with the drug polymer.

Drug-polymer coating **122** may include one or more drugs. Each drug may include a bioactive agent. The bioactive agent may be a pharmacologically active drug or bioactive compound. The bioactive agent may be eluted from the drug-polymer coating when the stent has been deployed in the body. Alternatively, drug-polymer coating **122** may comprise a bioerodible polymer that erodes after deployment in the body, releasing bioactive agents, drugs and other pharmaceutical agents. Elution refers to the transfer of the bioactive agent out from drug-polymer coating **122**. The elution rate is determined by the rate at which the bioactive agent is excreted from drug-polymer coating **122** into the body. The composition of the drug-polymer coating and the interdispersed drugs may control the elution rate of the bioactive agent.

The drug-polymer coating may be subject to degradation during processing, packaging, sterilization, or storage of a drug-polymer coated stent. During sterilization, for example, oxidation of the drug or polymer may occur, resulting in hydrolytic damage, cleavage of the polymeric bonds, breakdown of the polymer and/or drug, or actual cracking or peeling of the drug-polymer coating. Temperature excursions of the in-process or processed stent may incur delamination of all or a portion of the drug-polymer coating. The present invention solves this problem through the use of an adhesion promoter in either the drug polymer or an adhesion layer between the polymer-drug coating and the metallic stent so as to reduce or prevent drug-polymer delamination.

Upon insertion of catheter **110** and stent **120** with drug-polymer coating **122** into a directed vascular region of a human body, stent **120** may be expanded by applying pressure to a suitable balloon inside the stent, or by retracting a sheath to allow expansion of a self-expanding stent. Balloon deployment of stents and self-expanding stents are well known in the art. Catheter **110** may include a balloon used to expand stent **120.** Catheter **110** may include a sheath that retracts to allow expansion of the stent.

The adhesion promoter may be interdispersed within drug-polymer coating **122** or within an adhesion coating on the stent, and also may be eluted then metabolized or discarded by the body.

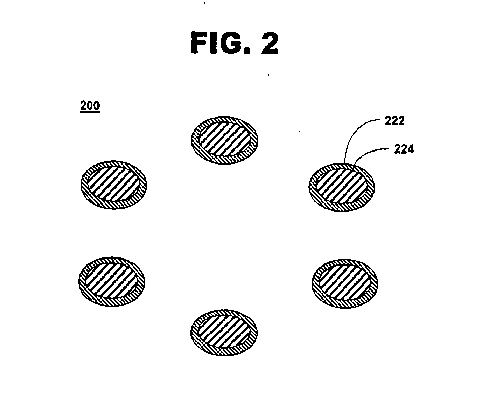

**FIG. 2** shows an illustration of a stent cross-section including a drug polymer and an activated adhesion promoter on the stent surface, in accordance with the present invention at **200**. Drug-polymer coated stent **200** with an adhesion promoter may include a drug-polymer coating **222** on a stent framework **224**. Drug-polymer coating **222** may contain one or more pharmaceutical drugs. Drug-polymer coating **222** may contain a polymeric matrix in which the adhesion promoter is interdispersed. The adhesion promoter may be interdispersed within drug-polymer coating **222.**

The drugs and the adhesion promoter may be encapsulated in a polymer coating using a microbead, microparticle or nanoencapsulation technology with albumin, liposome, ferritin or other biodegradable proteins and phospholipids, prior to application on the stent.

Stent framework **224** may include a metallic or polymeric base. Stent framework **224** may include a base material of stainless steel, nitinol, tantalum, an MP35N alloy, platinum, or titanium. The stent or stent framework may include a base material of a suitable biocompatible alloy, a suitable biocompatible material including a biodegradable polymeric material, or a combination thereof.

The bioactive agent may include an antineoplastic agent such as triethylene thiophosphoramide, an antiproliferative agent, an antisense agent, an antiplatelet agent, an antithrombogenic agent, an anticoagulant, an antibiotic, an anti-inflammatory agent, a gene therapy agent, an organic drug, a pharmaceutical compound, a recombinant DNA product, a recombinant RNA product, a collagen, a collagenic derivative, a protein, a protein analog, a saccharide, a saccharide derivative, or combinations thereof.

The bioactive agent may be any therapeutic substance that provides a therapeutic characteristic for the prevention and treatment of disease or disorders. An antineoplastic agent may prevent, kill, or block the growth and spread of cancer cells in the vicinity of the stent. An antiproliferative agent may prevent or stop cells from growing. An antisense agent may work at the genetic level to interrupt the process by which disease-causing proteins are produced. An antiplatelet agent may act on blood platelets, inhibiting their function in blood coagulation. An antithrombogenic agent may actively retard blood clot formation. An anticoagulant may delay or prevent blood coagulation with anticoagulant therapy, using compounds such as heparin and coumarins. An antibiotic may kill or inhibit the growth of microorganisms and may be used to combat disease and infection. An anti-inflammatory agent may be used to counteract or reduce inflammation in the vicinity of the stent. A gene therapy agent may be capable of changing the expression of a person's genes to treat, cure or ultimately prevent disease. An organic drug may be any small-molecule therapeutic material. A pharmaceutical compound may be any compound that provides a therapeutic effect. A recombinant DNA product or a recombinant RNA product may include altered DNA or RNA genetic material. Bioactive agents of pharmaceutical value may also include collagen and other proteins, saccharides, and their derivatives.

For example, the bioactive agent may be selected to inhibit vascular restenosis, a condition corresponding to a narrowing or constriction of the diameter of the bodily lumen where the stent is placed. The bioactive agent may generally control cellular proliferation. The control of cell proliferation may include enhancing or inhibiting the growth of targeted cells or cell types.

The bioactive agent may be an agent against one or more conditions including coronary restenosis, cardiovascular restenosis, angiographic restenosis, arteriosclerosis, hyperplasia, and other diseases and conditions. For example, the bioactive agent may be selected to inhibit or prevent vascular restenosis, a condition corresponding to a narrowing or constriction of the diameter of the bodily lumen where the stent is placed. The bioactive agent may generally control cellular proliferation. The control of cell proliferation may include enhancing or inhibiting the growth of targeted cells or cell types.

The bioactive agent may include podophyllotoxin, etoposide, camptothecin, a camptothecin analog, mitoxantrone, rapamycin, and their derivatives or analogs. Podophyllotoxin is an organic, highly toxic drug that has antitumor properties and may inhibit DNA synthesis. Etoposide is an antineoplastic that may be derived from a semi-synthetic form of podophyllotoxin to treat monocystic leukemia, lymphoma, small-cell lung cancer, and testicular cancer. Camptothecin is an anticancer drug that may function as a topoisomerase inhibitor. Related in structure to camptothecin, a camptothecin analog such as aminocamptothecin may be used as an anticancer drug. Mitoxantrone is also an important anticancer drug, used to treat leukemia, lymphoma, and breast cancer. Rapamycin or sirolimus is a medication that may interfere with the normal cell growth cycle and may be used to reduce restenosis. The bioactive agent may also include analogs and derivatives of these agents. Antioxidants may be beneficial on their own rights for their antirestonetic properties and therapeutic effects.

Drug-polymer coating **222** may soften, dissolve or erode from drug-polymer coated stent **200** to elute at least one bioactive agent. This elution mechanism may be referred to as surface erosion where the outside surface of the drug-polymer coating dissolves, degrades, or is absorbed by the body; or bulk erosion where the bulk of the drug-polymer coating biodegrades to release the bioactive agent. Eroded portions of the drug-polymer coating may be absorbed by the body, metabolized, or otherwise expelled.

The pharmaceutical drug may separate within drug-polymer coating **222** and elute the bioactive agent. Alternatively, the pharmaceutical drug may erode from drug-polymer coated stent **200** and then separate into the bioactive agent. The pharmaceutical drug or agent may be eluted and absorbed or expelled by the body. Drug-polymer coating **222** may include a single bioactive agent or multiple pharmaceutical drugs, in addition to the adhesion promoter.

Drug-polymer coating **222** may also include a polymeric matrix. For example, the polymeric matrix may include a caprolactone-based polymer or copolymer, or various cyclic polymers. The polymeric matrix may include various synthetic and non-synthetic or naturally occurring macromolecules and their derivatives. The polymeric matrix may include biodegradable polymers such as polylactide (PLA), polyglycolic acd (PGA) polymer, poly (e-caprolactone) (PCL), polyacrylates, polymethacryates, or other copolymers. The pharmaceutical drug may be dispersed throughout the polymeric matrix. The pharmaceutical drug or the bioactive agent may diffuse out from the polymeric matrix to elute the bioactive agent. The pharmaceutical drug may diffuse out from the polymeric matrix and into the biomaterial surrounding the stent. The bioactive agent may separate from within drug-polymer coating **222** and diffuse out from the polymeric matrix into the surrounding biomaterial.

The polymeric matrix may be selected to provide a desired elution rate of the bioactive agent. The pharmaceutical drugs may be synthesized such that a particular bioactive agent may have two different elution rates. A bioactive agent with two different elution rates, for example, would allow rapid delivery of the pharmacologically active drug within twenty-four hours of surgery, with a slower, steady delivery of the drug, for example, over the next two to six months. The adhesion promoters may be selected to firmly secure the rapidly deployed bioactive agents and the slowly eluting pharmaceutical drugs to the stent framework.

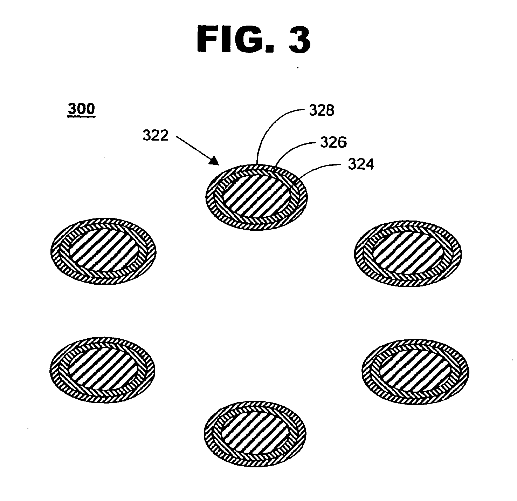

**FIG. 3** shows an illustration of a stent cross-section comprising a polymeric coating containing a drug-polymer coating disposed on an adhesion coating between the drug-polymer coating and the stent framework, in accordance with another embodiment of the present invention at **300**. Drug-polymer coated stent **300** with polymeric coating **322** includes an adhesion layer **326** on a stent framework **324** and a drug-polymer coating **328** on adhesion layer **326**. Adhesion layer **326** may be referred to herein as an adhesive coating. Drug-polymer coating **328** includes at least one interdispersed bioactive agent. Adhesion layer **326** may be void or nearly void of pharmaceutical drugs.

Adhesion layer **326** comprises activated adhesion promoter in accordance with the invention and serves to improve the adhesion and minimize the likelihood of delamination of the polymeric coating from stent framework **324.**

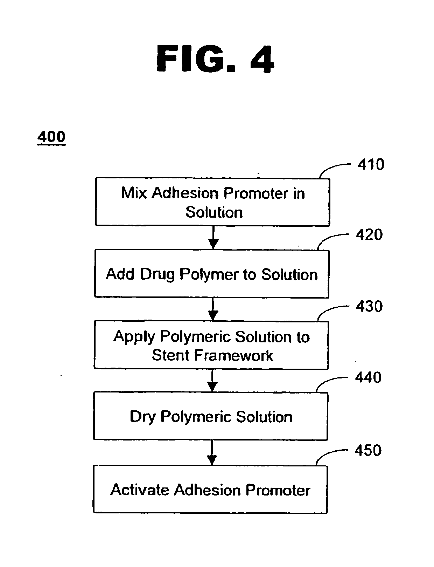

Another aspect of the current invention is a method of manufacturing a drug-polymer coated stent with an adhesion promoter. **FIG. 4** shows a flow diagram of one embodiment of a method for manufacturing a drug-polymer coated stent including an activated adhesion promoter, in accordance with the present invention at **400.**

The drug-polymer coated stent with an activated adhesion promoter is manufactured by mixing the activatable adhesion promoter in a polymeric solution, as seen at block **410.** Water, an alcohol such as methanol or ethanol, or other suitable solvents may be used to form the solution. The polymeric solution may also comprise a drug polymer.

A drug polymer may be added to the polymeric solution, as seen at block **420**. The drug polymer may include a polymeric matrix and one or more therapeutic compounds. Alternatively, the drug polymer may be mixed with a suitable solvent to form a polymeric solution, and the activatable adhesion promoter is then added to the polymeric solution.

To form a drug-polymer coating, a polymer such as a vinyl acetate derivative may be mixed with other polymers or monomers in a solvent such as isopropyl alcohol and added to the polymeric solution. The mixture may be reacted to form new polymers or modify existing polymers in the polymeric solution. One or more bioactive agents may be mixed with the polymerized mixture to form a drug polymer with a predefined elution rate. A suitable bioactive agent or a solution containing the bioactive agent may be mixed in with the polymeric solution up to 75 percent bioactive agent or greater by weight in the drug-polymer coating. Alternatively, a polymer such as a copolyester or block copolymer may be dissolved in a suitable solvent, and one or more bioactive agents may be added to the mixture. The mixture may be combined with the adhesion promoter in the polymeric solution.

The polymeric solution is applied to stent framework, as seen at block **430.** The polymeric solution may be applied to the stent by dipping, spraying, painting, brushing or any other suitable method for applying the polymer solution.

Excess liquid may be blown off and the film dried, as seen at block **440.** Drying of the polymeric solution to eliminate or remove any volatile components may be done at room temperature or elevated temperatures under dry nitrogen or other suitable environment. A second dipping and drying step may be used to thicken the coating. The thickness of the drug-polymer coating may range between 1.0 microns and 200 microns or greater in order to provide satisfactory pharmacological benefit with the bioactive agent. This action may provide a coating on the stent, although it may not provide sufficient energy to effect ring opening of the adhesion promoter and thus provide effective bond sites to the metal substrate.

The adhesion promoter may be activated after the drying step, as seen at block **450.** The polymer in contact with the metal may be supplied activation energy to activate the adhesion promoter. Activation energy may be supplied to the chemical structure of the adhesion promoter to provide a source of bonding to the metallic base of the stent. The adhesion promoter may be activated by heating the coated stent to an elevated temperature. Alternatively, the coated stent may be heated to nearly the melting temperature of the polymeric coating, or to at least a suitable temperature for breaking at least one of the organic rings in the adhesion promoter to increase the adhesive strength. The coated stent may be irradiated with laser radiation at one or more prescribed wavelengths to selectively break one of the bonds in the adhesion promoter. The adhesion promoter may be activated, for example, by exposing the coated stent to an x-ray irradiation source.

The adhesion promoter may be activated by heating the metallic stent framework. The polymer in contact with the metal may be supplied activation energy to form a bond on the stent. Induction heating, for example, will heat the stent metal, which in turn will heat the adhesion promoter in contact with the metal, thereby breaking the organic rings and increasing the adhesive strength of the polymeric adhesion promoter. Other methods of heating the metallic stent or of energy-activating the adhesive coating may be employed, such as laser irradiation, x-ray irradiation, or any other suitable method for activating the adhesion promoter. The adhesion promoter may be activated using an activation system such as an induction heater, an oven, a laser irradiation system, an x-ray irradiation system, or any other suitable activation system.

The coated stent may be integrated into a system for treating vascular conditions such as heart disease by assembling the coated stent onto a catheter. Finished coated stents may be reduced in diameter and placed into the distal end of the catheter, formed with an interference fit that secures the stent onto the catheter. The catheter with the stent may be placed in a catheter package and sterilized prior to shipping and storing. Sterilization using conventional medical means occurs before clinical use.

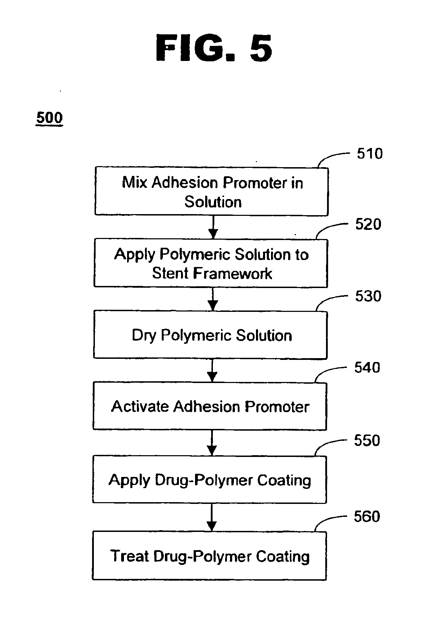

Another aspect of the current invention is a method of manufacturing a drug-polymer coated stent with a drug-polymer coating disposed on top of an adhesion layer. **FIG. 5** shows a flow diagram of one embodiment of a method for manufacturing a drug-polymer coated stent with an energy-activated adhesion coating and a drug-polymer coating, in accordance with the current invention at **500.**

The drug-polymer coated stent may be manufactured by mixing the activatable adhesion promoter in a solution, as seen at block **510**. An alcohol such as methanol or ethanol, water, or other suitable solvents may be used to form the polymeric solution.

The polymeric solution is applied to a metallic stent or a metallic stent framework, as seen at block **520.** The polymeric solution may be applied to the stent framework by dipping, spraying, painting, brushing, or by other suitable methods. The polymeric solution disposed on the metallic stent framework is dried, as seen at block **530**. Excess liquid may be blown off and the film dried to form an adhesion coating. Additional application and drying steps may be included to reach the desired thickness of the adhesion coating.

The adhesion promoter may be activated after the drying step, as seen at block **540**. The polymer in contact with the metal may be supplied with sufficient activation energy. The adhesion promoter on the coated stent or the metallic stent framework is activated by using any suitable energy-activation system such as an induction heater, an oven, a laser irradiation system, or an x-ray irradiation system. The coated stent may be heated to a prescribed temperature, for example, for breaking open at least one of the organic rings in the adhesion promoter to increase the adhesive strength. In another example, the coated stent may be irradiated with a laser operating at a suitable wavelength for breaking select bonds of organic rings in the adhesion promoter.

A drug-polymer coating is applied to the adhesion layer comprising the activated adhesion promoter, as seen at block **550.** The drug polymer may be mixed in a suitable solvent, and applied to the adhesion layer using an application technique such as dipping, spraying, painting or brushing. In subsequent coating operations, for example, a drug-polymer coating with olefinic polymers may adhere well over an adhesion layer comprising olefinic polymers.

The drug-polymer coating may be treated, as seen at block **560.** Treatment of the drug-polymer coating may include air drying or low-temperature heating in air, nitrogen, or other controlled environment. The drug-polymer coating may be treated by heating the drug-polymer coating to a predetermined temperature.

The coated stent with the drug-polymer coating disposed on the energy-activated adhesion layer may be coupled to a catheter.

## Claims

1. A polymer-coated biomedical device **characterised in that** an activated adhesion promoter comprising a ring-opened copolymer of maleic anhydride and an olefin is present within the polymer coating or between said coating and said device.

2. A biomedical device as claimed in claim 1 which is a drug-polymer coated stent.

3. A biomedical device as claimed in claim 2 wherein said stent has a metallic framework.

4. A biomedical device as claimed in claim 3 wherein said framework comprises stainless steel, nitinol, tantalum, MP35N or other biocompatible alloy, platinum or titanium.

5. A biomedical device as claimed in any preceding claim wherein the activated adhesion promoter comprises a ring-opened copolymer of maleic anhydride and ethylene.

6. A biomedical device as claimed in any of claims 2 to 5 wherein said stent is coupled to a catheter to provide a system for treating a vascular condition.

7. A method of preparing a polymer-coated biomedical device
**characterised in that** an activatable adhesion promoter comprising a copolymer of maleic anhydride and an olefin is incorporated into the polymer coating or between said coating and said device and is thereafter activated to effect ring opening of the copolymer.

8. A method as claimed in claim 7 wherein the biomedical device is a stent.

9. A method as claimed in claim 7 or claim 8 wherein the polymer coating comprises a drug-polymer.

10. A method as claimed in any of claims 7 to 9 wherein the adhesion promoter comprise a copolymer of maleic anhydride and ethylene.

11. A method as claimed in any of claims 7 to 10 wherein the adhesion promoter is activated using an induction heater, an oven, a laser irradiation system or an X-ray irradiation system.

12. A method as claimed in any of claims 7 to 11 wherein the coating is applied by dipping, spraying, painting or brushing.

13. A method as claimed in any of claims 7 to 12 wherein the polymer coating is applied to activated adhesion promoter disposed on the surface of the device.

## Patentansprüche

1. Polymerbeschichtete biomedizinische Vorrichtung, **dadurch gekennzeichnet, dass** ein aktivierter Haftvermittler mit einem Copolymer mit geöffnetem Ring aus Maleinsäureanhydrid und einem Olefin in der Polymerbeschichtung oder zwischen der Beschichtung und der Vorrichtung vorhanden ist.

2. Biomedizinische Vorrichtung nach Anspruch 1, die ein wirkstoff-polymerbeschichteter Stent ist.

3. Biomedizinische Vorrichtung nach Anspruch 2, wobei der Stent einen metallischen Rahmen hat.

4. Biomedizinische Vorrichtung nach Anspruch 3, wobei der Rahmen rostfreien Stahl, Nitinol, Tantal, MP35N oder eine andere biokompatible Legierung, Platin oder Titan umfasst.

5. Biomedizinische Vorrichtung nach einem der voranstehenden Ansprüche, wobei der aktivierte Haftvermittler ein Copolymer mit geöffnetem Ring aus Maleinsäureanhydrid und Ethylen umfasst.

6. Biomedizinische Vorrichtung nach einem der Ansprüche 2 bis 5, wobei der Stent mit einem Katheter gekoppelt ist, um ein System zur Behandlung eines Gefäßleidens bereitzustellen.

7. Verfahren zum Vorbereiten einer polymerbeschichteten biomedizinischen Vorrichtung, **dadurch gekennzeichnet, dass** ein aktivierbarer Haftvermittler mit einem Copolymer aus Maleinsäureanhydrid und einem Olefin in die Polymerbeschichtung oder zwischen die Beschichtung und die Vorrichtung eingebracht wird und danach aktiviert wird, um eine Ringöffnung des Copolymers zu bewirken.

8. Verfahren nach Anspruch 7, wobei die biomedizinische Vorrichtung ein Stent ist.

9. Verfahren nach Anspruch 7 oder Anspruch 8, wobei die Polymerbeschichtung ein Wirkstoffpolymer umfasst.

10. Verfahren nach einem der Ansprüche 7 bis 9, wobei der Haftvermittler ein Copolymer aus Maleinsäureanhydrid und Ethylen umfasst.

11. Verfahren nach einem der Ansprüche 7 bis 10, wobei der Haftvermittler unter Verwendung einer Induktionsheizung, eines Ofens, einem Laserbestrahlungssystem oder einem Röntgenstrahlenbestrahlungssystem aktiviert wird.

12. Verfahren nach einem der Ansprüche 7 bis 11, wobei die Beschichtung durch Eintauchen, Besprühen, Bemalen oder Bepinseln aufgebracht wird.

13. Verfahren nach einem der Ansprüche 7 bis 12, wobei die Polymerbeschichtung auf den aktivierten Haftvermittler, der auf der Oberfläche der Vorrichtung aufgetragen ist, aufgebracht wird.

## Revendications

1. Dispositif biomédical revêtu de polymère **caractérisé en ce qu'**un promoteur d'adhérence activé comprenant un copolymère à cycle ouvert d'anhydride maléique et une oléfine est présent dans le revêtement de polymère ou entre ledit revêtement et ledit dispositif.

2. Dispositif biomédical selon la revendication 1, qui est une endoprothèse vasculaire revêtue de polymère contenant une substance médicamenteuse.

3. Dispositif biomédical selon la revendication 2, dans lequel ladite endoprothèse vasculaire a une armature métallique.

4. Dispositif biomédical selon la revendication 3, dans lequel ladite armature comprend de l'acier inoxydable, du nitinol, du tantale, du MP35N ou un autre alliage biocompatible, du platine ou du titane.

5. Dispositif biomédical selon l'une quelconque des revendications précédentes, dans lequel le promoteur d'adhérence activé comprend un copolymère à cycle ouvert d'anhydride maléique et d'éthylène.

6. Dispositif biomédical selon l'une quelconque des revendications 2 à 5, dans lequel ladite endoprothèse vasculaire est couplée à un cathéter pour former un système pour le traitement d'une maladie vasculaire.

7. Procédé de préparation d'un dispositif biomédical revêtu de polymère **caractérisé en ce qu'**un promoteur d'adhérence activable comprenant un copolymère d'anhydride maléique et une oléfine est incorporé dans le revêtement de polymère ou entre ledit revêtement et ledit dispositif et est ci-après activé pour effectuer une ouverture de cycle du copolymère.

8. Procédé selon la revendication 7, dans lequel le dispositif biomédical est une endoprothèse vasculaire.

9. Procédé selon la revendication 7 ou la revendication 8, dans lequel le revêtement de polymère comprend un polymère contenant une substance médicamenteuse.

10. Procédé selon l'une quelconque des revendications 7 à 9, dans lequel le promoteur d'adhérence comprend un copolymère d'anhydride maléique et d'éthylène.

11. Procédé selon l'une quelconque des revendications 7 à 10, dans lequel le promoteur d'adhérence est activé en utilisant un appareil de chauffage à induction, un four, un système d'irradiation laser ou un système d'irradiation aux rayons X.

12. Procédé selon l'une quelconque des revendications 7 à 11, dans lequel le revêtement est appliqué par immersion, pulvérisation, peinture ou brossage.

13. Procédé selon l'une quelconque des revendications 7 à 12, dans lequel le revêtement de polymère est appliqué sur le promoteur d'adhérence activé disposé sur la surface du dispositif.
